(19) Europäisches Patentamt
European Patent Office
Office européen des brevets

(11) EP 2 377 398 A1

(12) # EUROPEAN PATENT APPLICATION
published in accordance with Art. 153(4) EPC

(43) Date of publication:
**19.10.2011 Bulletin 2011/42**

(21) Application number: **09833520.1**

(22) Date of filing: **21.12.2009**

(51) Int Cl.:
***A01N 43/90*** *(2006.01)* ***A01N 63/04*** *(2006.01)*
***A01P 7/04*** *(2006.01)* ***A61K 31/529*** *(2006.01)*
***A61P 33/14*** *(2006.01)* ***C07D 471/22*** *(2006.01)*
***C07D 491/22*** *(2006.01)*

(86) International application number:
**PCT/JP2009/071257**

(87) International publication number:
**WO 2010/071219 (24.06.2010 Gazette 2010/25)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **19.12.2008 JP 2008323882**
**27.03.2009 JP 2009078607**

(71) Applicant: **Meiji Seika Pharma Co., Ltd.**
**Tokyo 104-8002 (JP)**

(72) Inventors:
- **KURIHARA Kenichi**
  **Yokohama-shi**
  **Kanagawa 222-8567 (JP)**
- **HORIKOSHI Ryo**
  **Yokohama-shi**
  **Kanagawa 222-8567 (JP)**
- **NOMURA Masahiro**
  **Yokohama-shi**
  **Kanagawa 222-8567 (JP)**
- **NAKAMURA Satoshi**
  **Yokohama-shi**
  **Kanagawa 222-8567 (JP)**
- **HAYASHIMOTO Mizuki**
  **Yokohama-shi**
  **Kanagawa 222-8567 (JP)**
- **TSUCHIDA Mariko**
  **Yokohama-shi**
  **Kanagawa 222-8567 (JP)**
- **MINOWA Nobuto**
  **Yokohama-shi**
  **Kanagawa 222-8567 (JP)**

(74) Representative: **Tetaz, Franck Claude Edouard et al**
**Cabinet Regimbeau**
**139, rue Vendôme**
**69477 Lyon Cedex 06 (FR)**

(54) **16-KETOASPERGILLIMIDE AND PEST EXTERMINATING AGENT COMPRISING THE SAME AS ACTIVE INGREDIENT**

(57) Disclosed is a composition for use in controlling ectoparasites, comprising as an active ingredient at least one of compounds represented by formula (I) or (III) or salts thereof. The present invention provides a composition for use in controlling ectoparasites that has excellent ectoparasite control effect and is highly safe.



Printed by Jouve, 75001 PARIS (FR)

(Cont. next page)

[Chemical formula 1]

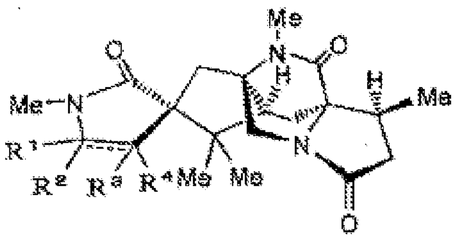

(I)

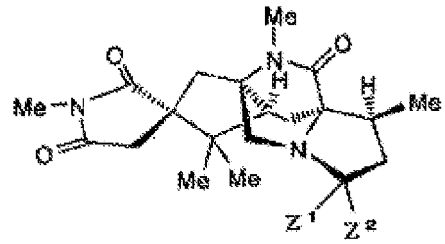

(III)

## Description

[CROSS-REFERENCE TO RELATED APPLICATIONS]

The application is based upon and claims the benefit of priority from the prior Japanese Patent Application No. 323882/2008, filed on December 19, 2008, and No. 78607/2009, filed on March 27, 2009; the entire contents of which are incorporated herein by reference.

[BACKGROUND OF THE INVENTION]

Field of Invention

The present invention relates to 16-keto aspergillimides having potent insecticidal activity and a composition for use in controlling ectoparasites that comprises the same as an active ingredient.

Background Art

16-Keto aspergillimide is a compound having the following structure that is reported as a metabolitic product produced by an organism belonging to Aspergillus and is known as an anthelmintically active substance against larvae at the third instar born of Haemonchus contortus which is a parasitic nematode in a digestive tract of mammals (The Journal of Antibiotics, 1997, 50(10), 840-846 (Non-patent Document 1)). However, there is no report about the activity of this substance against ectoparasites.

[Chemical formula 1]

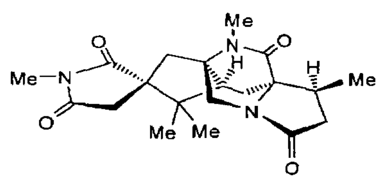

16-Keto aspergillimide

Further, Tetrahedron Letters, 1997, 38 (32), 5655-5658 (Non-patent Document 2), Bioscience, Biotechnology and Biochemistry, 2000, 64 (1), 111-115 (Non-patent Document 3), and Japanese Patent Application Laid-Open Publication No. 245383/1998 (Patent Document 1) report that asperparalines which are substances analogous to 16-keto aspergillimide have stupefacient activity against silk worms or insecticidal activity against beet armyworm (Spodoptera exigua), brown planthopper (Nilaparvata lugens (Stal)) and cockroach (Non-patent Documents 2 and 3 and Patent Document 1). They, however, do not report at all that this substance has ectoparasiticidal activity. Further, there is no report about a novel compound obtained by chemical conversion based on a mother nucleus constituting the substance, leading to an interest in novel derivatives and harmful organism control effect thereof.

[Chemical formula 2]

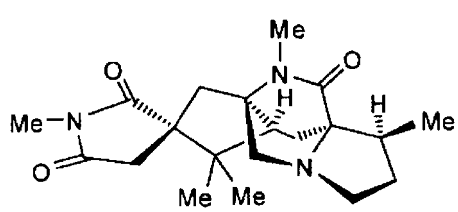

Asperparaline A

Paraherquamides having a structure similar to 16-keto aspergillimide and asperparaline A have control effect against endoparasite of mammals but are not known to have ectoparasiticidal activity. However, there is a report about high toxicity of paraherquamides (Journal of veterinary pharmacology and therapeutics, 2002, 25(4), 241-250 (Non-patent Document 4)).

[Chemical formula 3]

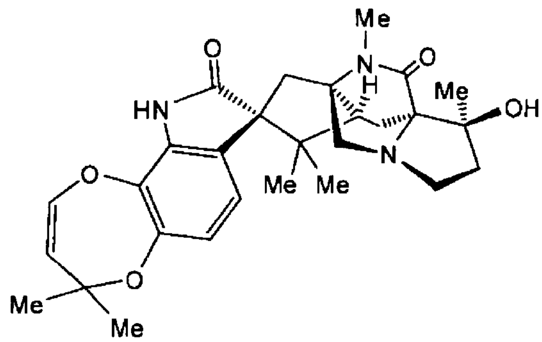

Paraherquamide A

Some of currently used ectoparasite control agents are highly toxic against mammals (The Pesticide Manual, 14th edition, published by The British Crop Protection Council) (Non-patent Document 5). Accordingly, novel ectoparasite control agents having high safety against mammals are desired.

Up to now, a number of harmful organism control agents are reported. However, the presence of insect pest species having drug resistance and hardly controllable insect pest species has been confirmed. Further, these harmful organism control agents involve problems such as safety against man and beast.

[PRIOR ART REFERENCES]

[PATENT DOCUMENTS]

[Patent Document 1] Japanese Patent Application Laid-Open Publication No. 245383/1998

[NON-PATENT DOCUMENTS]

[Non-patent Document 1] The Journal of Antibiotics,1997,50(10),840-846
[Non-patent Document 2] Tetrahedron Letters,1997,38(32),5655-5658
[Non-patent Document 3] Bioscience, Biotechnology and Biochemistry,2000,64(1),111-115
[Non-patent Document 4] Journal of veterinary pharmacology and therapeutics, 2002, 25(4),241-250
[Non-patent Document 5] The Pesticide Manual, 14th edition, published by The British Crop Protection Council

[SUMMARY OF THE INVENTION]

The present inventors have now found that the use of at least one of compounds represented by formula (I) or (III) or salts thereof as an active ingredient can realize potent ectoparasiticidal effect and high safety, particularly potent ectoparasiticidal effect against homeothermic animals.

Further, the present inventors have now found that the use of at least one of compounds reprenseted by formula (I) or salts thereof can realize potent harmful organism control effect, penetrative transferable insecticidal effect, and high safety, particularly harmful organism control effect against homeothermic animals. The present invention has been made based on such finding.

Accordingly, an object of the present invention is to provide a composition for use in controlling ectoparasites

that are safe to use, or a compound having harmful organism control effect or a salt thereof.

According to one aspect of the present invention, there is provided a composition for use in controlling ectoparasites, comprising as an active ingredient at least one of compounds represented by formula (I) or salts thereof:

[Chemical formula 4]

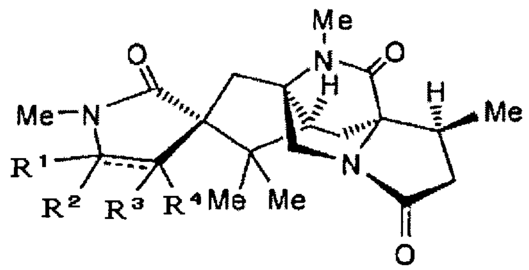

(I)

wherein
$R^1$ represents
a hydrogen atom,
hydroxyl,
optionally substituted $C_{1-10}$ alkyloxy (preferably methoxy),
optionally substituted $C_{7-15}$ aralkyloxy (preferably benzyloxy), optionally substituted $C_{1-10}$ (preferably$C_{1-6}$, more preferably $C_{1-4}$)
alkylcarbonyloxy (more preferably acetyloxy), or
optionally substituted $C_{7-15}$ aralkylcarbonyloxy,
$R^2$ represents a hydrogen atom, or
$R^1$ and $R^2$ together represent oxo,
$R^3$ represents
a hydrogen atom,
hydroxyl,
optionally substituted $C_{1-5}$ alkyl, or
optionally substituted $C_{7-15}$ aralkyl,
$R^4$ represents a hydrogen atom,
or when $R^1$ and $R^3$ combine together to represent a double bond and $R^2$ represents a hydrogen atom, $R^4$ represents formula (II)

[Chemical formula 5]

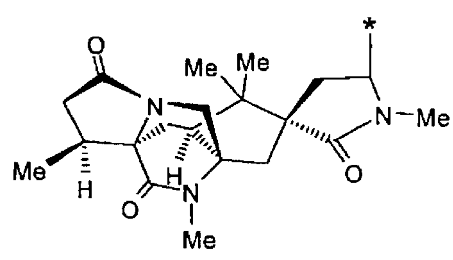

(II)

or when both $R^2$ and $R^4$ represent a hydrogen atom, $R^1$ and $R^3$ together form a cyclic ether, or $R^1$ and $R^3$ combine together to represent a double bond,
provided that
when $R^1$ represents hydroxyl,

optionally substituted $C_{1-10}$ alkyloxy,
optionally substituted $C_{7-15}$ aralkyloxy,
optionally substituted $C_{1-10}$ alkylcarbonyloxy, or
optionally substituted $C_{7-15}$ aralkylcarbonyloxy,
all of $R^2$, $R^3$ and $R^4$ then represent a hydrogen atom, or
when $R^1$ and $R^2$ together represent oxo, $R^3$ then represents hydroxyl, optionally substituted $C_{1-5}$ alkyl or optionally substituted $C_{7-15}$ aralkyl (preferably benzyloxy), or
compounds represented by formula (III) or salts thereof:

[Chemical formula 6]

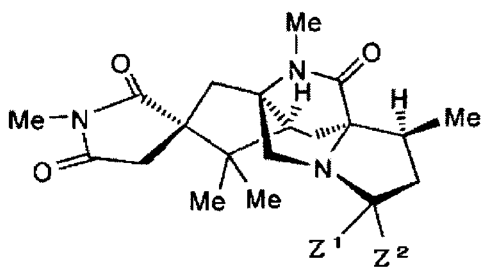

(III)

wherein $Z^1$ and $Z^2$ together represent oxo or each represent a hydrogen atom.

According to another aspect of the present invention, there is provided a method for controlling ectoparasites, comprising applying an effective amount of at least one of compounds represented by formula (I) or (III) or salts thereof to an animal.

According to still another aspect of the present invention, there are provided compounds represented by formula (I) or salts thereof.

According to a further aspect of the present invention, there is provided a composition for use in controlling harmful organisms, comprising as an active ingredient at least one of compounds represented by formula (I) or salts thereof.

According to another aspect of the present invention, there is provided a composition for agricultural and horticultural insect pests, comprising as an active ingredient at least one of compounds represented by formula (I) or salts thereof.

According to still another aspect of the present invention, there is provided a method for controlling agricultural and horticultural insect pests, the method comprising applying an effective amount of the above composition to an object selected from the group consisting of water surface, soil, nutrient solution in nutriculture, solid medium in nutriculture, and seed, root, tuber, bulb, and rhizome of a plant.

According to a further aspect of the present invention, there is provided a method for controlling agricultural and horticultural insect pests, the method comprising applying an effective amount of the above composition to plants.

[DETAILED DESCRIPTION OF THE INVENTION]

Definition

In the present specification, the number of carbon atoms in alkylcarbonyloxy or aralkylcarbonyloxy is expressed in terms of the number of carbon atoms in an alkyl or aralkyl moiety obtained by removing a carbonyloxy structure from the alkylcarbonyloxy or aralkylcarbonyloxy. Me represents methyl.

Alkyl and an alkyl moiety in an alkyl moiety-containing substituent (for example, alkyloxy, alkylcarbonyl, or aralkyl), unless otherwise specified, preferably have 1 to 6 carbon atoms ($C_{1-6}$) and, for exmaple, may be any of methyl, ethyl, propyl, butyl, pentyl, hexyl, isopropyl, isobutyl, tert-butyl, isopentyl, neopentyl, tert-pentyl, cyclopropyl, cyclobutyl, and cyclopentyl and the like that are of a straight chain type, a branched chain type, a cyclic type, or a combination thereof, preferably of a straight chain type or a branched chain type, more preferably methyl, ethyl, propyl, butyl, pentyl, hexyl and cyclopropylmethyl.

Aralkyl represents arylalkyl.

Aryl means ,unless otherwise specified, a heteroatom-free 6 to 14-membered (mono- to tricyclic) aromatic ring, for example, phenyl, naphthyl, biphenylyl, or phenanthryl. Here 6 to 14-membered aryl contains 6 to 14 carbon atoms in its ring system.

In the present specification, examples of preferred substituents in each group described as "optionally substituted" include halogens, $C_{1-4}$ alkyl optionally substituted by a halogen (excluding that the optionally substituted group is alkyl), $C_{1-4}$ alkyloxy optionally substituted by a halogen, nitro and cyano.

Compounds represented by formulae (1) and (III) and process for producing them

Salts of 16-keto aspergillimides represented by formulae (1) and (III) and their analogous compounds are agriculturally and zootechnically acceptable acid addition salts, and examples thereof include hydrochloride, nitrate, sulfate, phosphate, or acetate.

Examples of preferred compounds represented by formula (I) include compounds represented by formula (IV):

[Chemical formula 7]

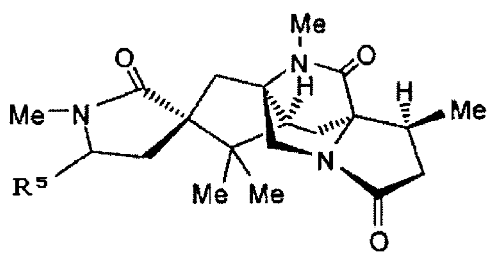

(IV)

wherein

$R^5$ represents

a hydrogen atom,

hydroxyl,

optionally substituted $C_{1-10}$ alkyloxy (preferably methoxy), optionally substituted $C_{7-15}$ aralkyloxy(preferably benzyloxy), optionally substituted $C_{1-10}$ (preferably$C_{1-6}$, more preferably $C_{1-4}$) alkylcarbonyloxy (more preferably acetyloxy), or optionally substituted $C_{7-15}$ aralkylcarbonyloxy).

Examples of another preferred compounds represented by formula (I) include compounds represented by formula (V):

[Chemical formula 8]

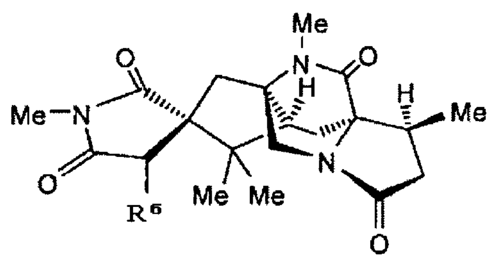

(V)

wherein $R^6$ represents hydroxyl, optionally substituted $C_{1-5}$ alkyl or optionally substituted $C_{7-15}$ aralkyl (preferably benzyl).

Examples of another preferred compounds represented by formula (I) include compounds represented by formula (VI):

[Chemical formula 9]

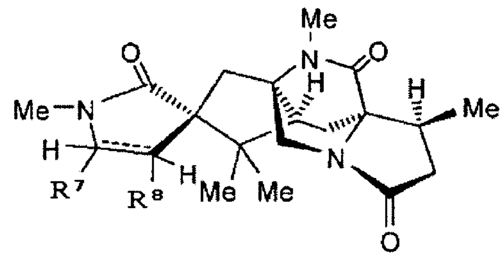

(VI)

wherein $R^7$ and $R^8$ together form a cyclic ether, or combine together to represent a double bond.

Still another preferred examples of compounds represented by formula (I) include compounds represented by formula (I) wherein
all of $R^1$, $R^2$, $R^3$ and $R^4$ then represent a hydrogen atom or
$R^1$ represents hydroxyl, and $R^2$, $R^3$ and $R^4$ represent a hydrogen atom or
$R^1$ and $R^2$ together represent oxo, $R^3$ represents hydroxyl, and $R^4$ represents a hydrogen atom or
$R^1$ and $R^2$ together represent oxo, $R^3$ represents benzyl, $R^4$ represents a hydrogen atom or
$R^1$ represents methoxy, $R^2$, $R^3$ and $R^4$ represent a hydrogen atom,
$R^1$ represents benzyloxy, and $R^2$, $R^3$ and $R^4$ represent a hydrogen atom or
$R^1$ represents methylcarbonyloxy, and $R^2$, $R^3$ and $R^4$ represent a hydrogen atom or
$R^1$ and $R^3$ combine together to represent a double bond, $R^2$ represents a hydrogen atom, and $R^4$ represents a group represented by formula (IV).

Examples of preferred 16-keto aspergillimide derivatives represented by formula (I) are shown in Table 1 below.

[Table 1]

| Compound No. | | Compound No. | |
|---|---|---|---|
| 1 | 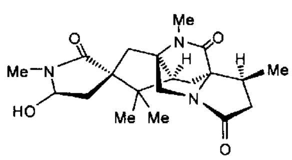 | 6 | 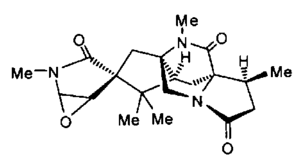 |
| 2 | 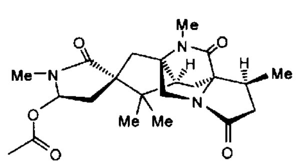 | 7 | 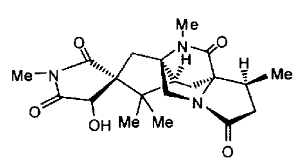 |
| 3 | 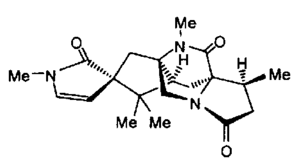 | 8 | 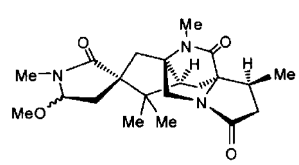 |

(continued)

| Compound No. | | | Compound No. | |
|---|---|---|---|---|
| 4 | 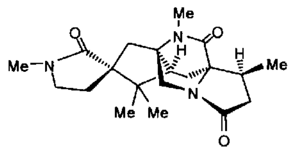 | | 9 | 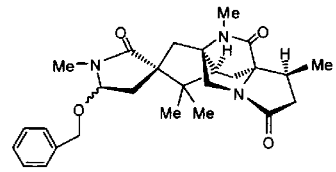 |
| 5 | 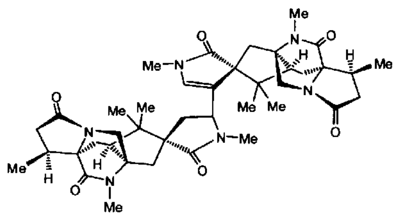 | | 10 | 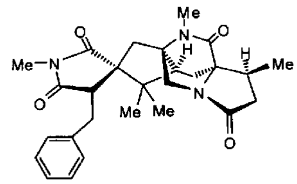 |

Specific examples of compounds analogous to 16-keto aspergillimides represented by formula (III) are shown in Table 2 below.

[Table 2]

| | | | | |
|---|---|---|---|---|
| A | 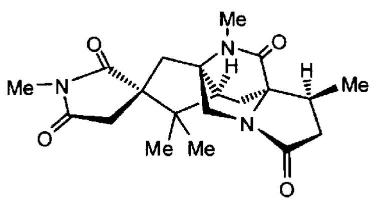 | | B | 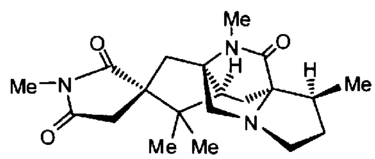 |

Compound A (16-keto aspergillimide) can be obtained from a cultured product of 16-keto aspergillimide producing fungi, for exmaple, according to a method described in The Journal of Antibiotics, 1997, 50 (10), 840-846 (Non-patent Document 1). Further, derivatives shown in Table 1 can be produced using compound A as a starting material.

Compound B (asperparaline A) can be obtained from a cultured product of asperparaline A producing fungi, for example, according to a method described in Japanese Patent Application Laid-Open Publication No. 245383/1998 (Patent Document 1).

The process for producing 16-keto aspergillimide derivatives represented by formula (I) will be described below in detail.

Compound 1 can be obtained by subjecting 16-keto aspergillimide to a reduction reaction. Suitable reagents usable in the reduction reaction include hydride reducing agents such as sodium borohydride ($NaBH_4$), lithium borohydride and tetrabutylammonium borohydride. Preferred is sodium borohydride. The reagent may be used in an amount of 1 to 5 equivalents based on 16-keto aspergillimide. Reaction solvents usable herein include alcohol solvent such as methanol and ethanol. When the compound as the substrate in this reaction is not dissolved in an alcohol solvent, the compound may be dissolved in tetrahydrofuran (THF) and the like to prepare a 1 : 1 mixed solution. The reaction proceeds at a temperature of -10 to 50˚C, and the reaction time is 1 min to 3 hr.

Compound 3 can be obtained as a result of the progress of an elimination reaction through compound 2 obtained by modifying hydroxyl of compound 1 with a suitable substituent. Regarding modification of hydroxyl in this reaction, modification with a substituent having a proper level of stability and leaving ability, preferably acyl such as acetyl, is suitable. A reagent used in this acylation reaction may be an acyl halide such as acetyl chloride or an acid anhydride such as acetic anhydride and is used in an amount of 1 to 10 equivalents. Bases usable in this reaction include organic bases such as pyridine and, further, collidine, lutidine, diethylamine, triethylamine, and isopropylamine. The reaction is accelerated by the addition of a catalytic amount of 4-dimethylaminopyridine (4-DMAP). The reaction solvent may be an aprotic solvent such as methylene chloride or chloroform used in a conventional acylation reaction. The reaction

proceeds well at a temperature of 0 to 60˚C, and the reaction time is 1 to 24 hr. The subsequent elimination reaction proceeds in silica gel in the purification.

Compound 5 can be obtained using trifluoromethanesulfonic acid anhydride as an activating agent of hydroxyl in compound 1. Preferably, the amount of trifluoromethanesulfonic anhydride used in this reaction is 1 to 5 equivalents based on compound 1. Bases usable in this reaction include organic bases such as pyridine and, further, collidine, lutidine, diethylamine, triethylamine and isopropylamine. The reaction solvent may be an aprotic solvent such as methylene chloride or chloroform. The reaction proceeds well at a temperature of -10 to 50˚C, and the reaction time is 1 min to 12 hr.

Compound 4 can be obtained by catalytic reduction of compound 3 in the presence of a palladium catalyst in a hydrogen atmosphere under applied pressure or ordinary pressure. The catalyst usable in this reaction may be supported palladium. Preferably, palladium carbon (Pd-C) (content 1 to 100%) is hydrogenated in a solvent under hydrogen atmosphere or by bubbling, and 1 to 100% by mole thereof is used. Alcohol solvents such as methanol and ethanol may be used as the reaction solvent, and the reaction proceeds well at a temperature of 10 to 60(C. The reaction time is 1 hr to 2 days.

Compound 6 can be obtained by subjecting compound 3 to an epoxidation reaction with an oxidation reagent. Oxidizing agents usable in this reaction include m-chloroperbenzoic acid (m-CPBA), and, further, aqueous hydrogen peroxide and peroxides such as peracetic acid. In some cases, the reaction is accelerated by the addition of sodium hydrogencarbonate. The reaction solvent may be methylene chloride, chloroform or an alcohol solvent such as methanol. Water may be added. The reaction proceeds at a temperature of -10 to 60(C, and the reaction time is 1 hr to 2 days.

Compound 7 can also be obtained by reacting compound 3 with potassium permanganate in the presence of metaperiodic acid. The amount of metaperiodic acid and potassium permanganate may be 1 to 20 equivalents based on compound 3. In addition to acetone, t-butanol and the like may be used as the reaction solvent. The same amount of water may also be added. The reaction proceeds at a temperature of -10 to 60(C, and the reaction time is 1 hr to 3 days.

Some novel basic skeleton or 16-keto aspergillimide thus produced can be modified by the following method. For example, hydroxyl in compound 1 can be modified with a substituent by alkylation or acylation, and alkyl can be introduced into (-position in an succinic acid imide carbonyl group in 16-keto aspergillimide (compounds 2, 8, 9 and 10).

The alkylation may be performed by a reaction with an alkyl halide or aralkyl halide containing desired alkyl in the presence of a base. An alkyl or aralkyl halides such as methyl iodide or benzyl bromide may be used in an amount of 1 to 10 equivalents as the reagent in the alkylation reaction. Bases usable in this reaction include sodium hydroxide, lithium hydride and metal alkoxide bases such as sodium methoxide and sodium ethoxide. Reaction solvents usable herein include aprotic polar solvents such as methylene chloride, chloroform, tetrahydrofuran, or dimethylformamide (DMF). A reaction proceeds at a temperature of 0 to 100(C, and the reaction time is 1 to 24hr.

The acylation may be performed by a reaction, for example, with an acyl halide containing desired alkyl in the presence of a base. Reagents usable in this acylation reaction include, for example, alkyl carbonyl or aralkyl carbonyl halides such as acetyl chloride or benzoyl chloride and, further, acid anhydrides such as acetic anhydride. The reagent may be used in an amount of 1 to 10 equivalents. Bases usable in this reaction include organic bases such as pyridine and, further, collidine, lutidine, diethylamine, triethylamine and isopropylamine. In some cases, the addition of a catalytic amount of 4-DMAP accelerates the reaction. The reaction solvent may be pyridine which serves also as a base, or an aprotic solvent such as methylene chloride or chloroform used in the conventional acylation reaction. The reaction proceeds at a temperature of 10 to 80˚C, and the reaction time is 1 hr to 2 days.

<u>Composition for use in controlling ectoparasites</u>

The composition for use in controlling ectoparasites according to the present invention comprises as an active ingredient at least one of compounds represented by formula (I) or (III) or salts thereof. The composition for use in controlling ectoparasites according to the present invention has potent ectoparasiticidal effect and is highly safe, particularly has potent ectoparasiticidal effect against homeothermic animals.

In a preferred embodiment of the present invention, the composition for use in controlling ectoparasites according to the present invention comprises as an active ingredient at least one of compounds represented by formula (III) or salts thereof.

Insect pest species against which the composition for use in controlling ectoparasites according to the present invention exhibits ectoparasiticidal effect include, but are not limited to, ectoparasites for animals, for example, Aphaniptera, for example, Ctenocephalides felis and Pulex irritans, Anoplura, for example, Pediculus humanus and Pediculus pubis, mites or ticks such as Boophilus spp., Haemaphysalis longicornis, Rhipicephalus sanguineus, Haemaphysalis flava, Sarcoptes scabiei, Dermanyssus gallinae, Ornithonyssus sylviarum, Ornithonyssus bacoti, and Leptotrombidium, horseflies, flies, for example, Lucilia spp., mosquitoes, for example, Stegomyia albopicta and Culex pipiens pallens, Simuliidae, and Ceratopogonidae. More preferred are Aphaniptera and mites or ticks.

Examples of preferred other ectoparasiticidal agents admixable into the composition for use in controlling

ectoparasites according to the present invention include pyrethroid compounds, neonicotinoid compounds, avermectin compounds, macrolide compounds, phenylpyrazole compounds, phenylpyrrole compounds, organophosphorus compounds, carbamate compounds, nereistoxin derivatives, organochlorine compounds, benzoyl urea compounds, juvenile hormone analogies, molting hormone analogies, spinosyn compounds, cyclodepsipeptide compounds, sodium channel blockers for nerve cells, insecticidal macrocyclic lactones, γ-aminobutyric acid (GABA) antagonists, ryanodine receptor agonistic compounds. Examples of more preferred other ectoparasiticidal agents include ivermectin, selamectin, moxidectin, doramectin, eprinomectin, milbemycin oxime, maduramycin, imidacloprid, dinotefuran, nitenpyram, acetamiprid, thiacloprid, thiamethoxam, clothianidin, sulfoxaflor, fipronil, ethiprole, pyriprole, allethrin, d·d-T allethrin, dl·d-T80 allethrin, pyrethrins, permethrin, phenothrin, flumethrin, cyfluthrin, d(d-T80 prarethrin, phthalthrin, transfluthrine, resmethrin, etofenprox, cyphenothrin, pyrethrum extract, piperonylbutoxide, synepirin222, synepirin 500, pyriproxyfene, lufenuron, methoprene, etoxazole, deet, diazinon, fenitrothion, dichlorvos, prothiofos, trichlofon, coumaphos, malathion, dimpylate, naled, tetrachlorvinphos, cythioate, metoxadiazon, carbaryl, fenobucarb, propoxur, diflubenzuron, teflubenzuron, cyromazine, triflumuron, star anise oil, amitraz, metaflumizone and spinosad. Examples of still more preferred other ectoparasiticidal agents include ivermectin, imidacloprid, nitenpyram, acetamiprid, thiamethoxam, clothianidin, dinotefuran and sulfoxaflor.

The composition for use in controlling ectoparasites according to the present invention is provided in a variety of dosage forms acceptable as ectoparasiticidal agents, for example, liquid formulations, sprays, foam formulations, tablets, granules, fine subtilaes, dust, capsules, tablets, chewable formulations, injections, suppositories, creams, shampoos, rinses, resin agents, fumigants and poison baits. Among them, liquid formulations are particularly preferred.

Carriers usable in the preparation of the composition for use in controlling ectoparasites according to the present invention include liquid carriers, solid carriers, gaseous carriers, surfactants, dispersants and other adjuvants for formulations.

Liquid carriers include, for example, alcohols such as methanol, ethanol, isopropanol, polyethylene glycol, propylene glycol, dipropylene glycol, tripropylene glycol, and glycerin; ketones such as acetone and methyl ethyl ketone; aromatic hydrocarbons such as benzyl alcohol, benzene, toluene, xylene, ethylbenzene, and methyl naphthalene, aliphatic hydrocarbons such as paraffin, n-hexane, cyclohexane, kerosine and kerosene, ethers such as diethylene glycol monoethyl ether, diethylene glycol monomethyl ether, diisopropyl ether, diethyl ether, dioxane, and tetrahydrofuran, esters such as propylene carbonate, ethyl acetate, butyl acetate, benzyl benzoate, isopropyl myristate, and fatty acid ester of propylene glycol, nitriles such as acetonitrile and isobutyronitrile, amides such as dimethylformamide, dimethylacetamide, and N-methylpyrrolidone, halogenated hydrocarbons such as dichloromethane, trichloroethane, and carbon tetrachloride, animal and vegetable oils such as soybean oils and cotton seed oils, dimethylsulfoxide, silicone oils, higher fatty acids, glycerol formal, and water.

Solid carriers include, for example, impalpable powders and particles of clays such as kaolin clay, diatomaceous earth, bentonite, and acid white clay, synthetic hydrous silicon oxide, talc, ceramic, other inorganic minerals such as selenite, quartz, sulfur, activated carbon, calcium carbonate, and hydrated silica, starch, lactose, and synthetic polymers such as vinyl chloride polymer and polyurethane.

Adjuvants for preparations such as conventional emulsifiers, dispersants, spreaders, wetting agents, suspending agents, preserving agents, and propellants and the like may be further incorporated in the liquid formulation. Further, conventional coating film forming agents may be incorporated. Surfactants for emulsification, dispersion, spreading or the like include, for example, soaps, polyoxyalkylene alkyl (aryl) ethers, polyoxyethylene alkyl allyl ethers, polyoxyethylene fatty acid esters, higher alcohols, and alkylarylsulfonates. Dispersants include, for example, casein, gelatin, polysaccharides, lignin derivatives, saccharides, and synthetic water soluble polymers. Spreaders/wetting agents include, for example, glycerin and polyethylene glycol. Susspending agents include, for example, casein, gelatin, hydroxypropylcellulose, and gum arabic. Stabilizers include, for example, phenolic antioxidants such as BHT and BHA, amine antioxidants such as diphenylamine, and organic sulfur antioxidants. Preserving agents include, for example, methyl p-oxybenzoate, ethyl p-oxybenzoate, propyl p-oxybenzoate, and butyl p-oxybenzoate. The carriers, surfactants, dispersants, and adjuvants may if necessary be used solely or in a combination of two or more. Perfumes, synergists and the like may also be incorporated. The content of the active ingredients in the composition for use in controlling ectoparasiticidaes according to the present invention is generally 1 to 75% by weight for the liquid formulation.

Carriers usable for the preparation of creams include, for example, nonvolatiel hydrocarbons such as liquid paraffin, lanolin hydrogenated fats and oils, higher fatty acids, fatty acid esters, animal and vegetable oils, silicone oils and water. The emulsifiers, humectant, antioxidants, perfumes, borax and ultraviolet absorbers may if necessary be used solely or in a combination of two or more. Emulsifiers include, for example, fatty acid sorbitan, polyoxyethylene alkyl ethers, and fatty acid polyoxyethylene. The content of the active ingredients in the composition for use in controlling ectoparasiticides according to the present invention is generally 0.5 to 70% by weight for the cream.

The capsules, pills or tablets may be used in such a manner that the active ingredients in the composition according to the present invention are divided into suitable small portions, the small portion is mixed with a diluting solution or a carrier such as starch, lactose, or talc, a disintegrator such as magnesium stearate and/or a binder is further

added thereto, and, if necessary, the mixture is tabletted.

Carriers for the preparation of injections should be prepared as an aseptic solution. The aseptic solution may contain other substances, for example, a salt or glucose in an amount enough to be isotonicated with blood. Carriers usable herein include organic solvents, for example, esters such as glycerides, benzyl benzoate, isopropyl myristate, and fatty acid derivatives of propylene glycol, N-methylpyrrolidone, and glycerol formal. The content of the active ingredients in the composition for use in controlling ectoparasiticides according to the present invention is generally 0.01 to 10% by weight for the injection.

Carriers usable for the preparation of resin agents include, for example, vinyl chloride polymers, and polyurethane. Plasticizers such as phthalic acid esters, adipic acid esters and stearic acid may be added to these bases. After kneading of the active ingredients according to the present invention into the base, the kneaded product is molded, for example, by injection molding, extrusion, or press molding. The molded product may also be further properly subjected to molding, cutting or the like to obtain an ear tag for animals or insecticidal collar for animals.

Carriers usable for toxic baits include bait substances and attractants, for example, farina such as wheat flour and corn flour, starch such as corn starch and potato starch, saccharides such as granulated sugar, malt sugar, and honey, food flavors such as glycerin, onion flavor, and milk flavor, animal powders such as pupal powder and fish powder, and various pheromones. The content of the active ingredients in the composition for use in controlling ectoparasiticides according to the present invention is generally 0.0001 to 90% by weight for the toxic bait.

The composition for use in controlling ectoparasites accoridng to the present invention can control ectoparasites, for example, by oral or injection administration into the body of an object animal, administration onto the whole or a part of the body surface of an object animal, covering of a place where invasion, parasitism, and migration of ectoparasites are expected, and application to animal-rearing houses.

The composition for use in controlling ectoparasites accoridng to the present invention as such may be used, or alternatively may be diluted with water, a liquid carrier, a commercially available shampoo, a rinse, a feed, an underlayment for animal-rearing houses or the like.

According to another aspect of the present invention, there is provided use of at least one of compounds represented by formula (I) or (III) or salts thereof as an ectoparasiticidal agent.

According to still another aspect of the present invention, there is provided a method for controlling ectoparasites, comprising applying an effective amount of at least one of compounds represented by formula (I) or (III) or salts thereof to an animal. Animals to which the composition for use in controlling ectoparasites according to the present invention is applied include, but are not limited to, preferably homeothermic animals, more preferably pigs, horses, cattle, sheep, goats, rabbits, chickens, domestic ducks, turkeys, mice, rats, guinea pigs, monkeys, dogs, cats and birds.

Composition for use in controlling harmful organisms

The composition for use in controlling harmful organisms according to the present invention comprises as an active ingredient at least one of compounds represented by formula (I) or salts thereof. The composition for use in controlling harmful organisms according to the present invention exhibits potent harmful organism control effect, penetrative transferable insecticidal effect, and is highly safe, particularly has harmful organism control effect against homeothermic animals.

Insect pest species against which the composition for use in controlling harmful organisms, comprising as an active ingredient at least one of compounds represented by formula (I) or salts thereof exhibits pesticidal effect include, but are not limited to, agricultural and horticultural insect pests, for example, lepidopteran insect pests, for example, Spodoptera litura, Mamestra brassicae, Pseudaletia separata, Pieris rapae, Plutella xylostella, Spodoptera exigua, Chilo suppressalis, Cnaphalocrocis medinalis, Tortricidae, Carposinidae, Lyonetiidae, Lymantriidae, Agrotis spp., Helicoverpa spp., and Heliothis spp.; hemipteran insect pests, for example, Aphididae, Adelgidae or Phylloxeridae such as Myzus persicae, Aphis gossypii, Aphis fabae, corn leaf aphid (Rhopalosiphum maidis), Acyrthosiphon pisum, Aulacorthum solani, Aphis craccivora, Macrosiphum euphorbiae, Macrosiphum avenae, Methopolophium dirhodum, Rhopalosiphum padi, Schizaphis graminum, Brevicoryne brassicae, Lipaphis erysimi, Aphis citricola, Rosy apple aphid, Eriosoma lanigerum, Toxoptera aurantii, and Toxoptera citricidus; Cicadellidae such as Nephotettix cincticeps, Delphacidae such as Laodelphax striatellus, Nilaparvata lugens, and Sogatella furcifera; heteropteran insect pests such as Eysarcoris ventralis, Nezara viridula, and Trigonotylus caelestialium; Aleyrodidae such as Bemisia tabaci, and Trialeurodes vaporariorum; Coccidea (Diaspididae, Margarodidae, Ortheziidae, Aclerdiae, Dactylopiidae, Kerridae, Pseudococcidae, Coccidae, Eriococcidae, Asterolecaniidae, Beesonidae, Lecanodiaspididae, or Cerococcidae), such as Pseudococcus comstocki and Planococcus citri; Coleopteran insect pests, for example, Lissorhoptrus oryzophilus, Callosobruchuys chienensis, Tenebrio molitor, Diabrotica virgifera virgifera, Diabrotica undecimpunctata howardi, Anomala cuprea, Anomala rufocuprea, Phyllotreta striolata, Aulacophora femoralis, Leptinotarsa decemlineata, Oulema oryzae, Bostrychidae, and Cerambycidae; Acari, for example, Tetranychus urticae, Tetranychus kanzawai, and Panonychus citri; Hymenopteran insect pests, for example, Symphyta; Orthopteran insect pests, for example, Acrididae; Dipteran insect pests, for example,

Muscidae and Agromyzidae; Thysanopteran insect pests, for example, Thrips palmi and Frankliniella occidentalis; Plant Parasitic Nematodes, for example, Meloidogynidae, Pratylenchidae, Aphelenchoides besseyi and Bursaphelenchus xylophilus; and zooparasites, for example, Siphonaptera, for example, Ctenocephalides felis and Pulex irritans, Anoplura, for example, Pediculus humanus and Pediculus pubis, zoobiotic mites or ticks such as Boophilus microplus., Haemaphysalis longicornis, Rhipicephalus sanguineus, Haemaphysalis flava, Sarcoptes scabiei, Dermanyssus gallinae, Ornithonyssus sylviarum, Ornithonyssus bacoti, and Leptotrombidium, horseflies, flies, for example, Lucilia spp., mosquitoes, for example, Stegomyia albopicta and Culex pipiens pallens, Simuliidae, Ceratopogonidae, Trematoda, Acanthocephala, Cestoda, Nematoda, Sarcomastigophora and Ciliata, and Sporozoa. More preferred are Hemipteran insect pests, Thysanopteran insect pests, Dipteran insect pests, Coleoptera insect pests, Aphaniptera and zoobiotic mites or tickes. Still more preferred are Hemipteran insect pests. Particularly preferred are Aphaniptera and zoobiotic mites or ticks.

Other harmful organism control agents admixable into compounds represented by formula (I) or salts thereof according to the present invention include insecticides, fungicides, miticides or tickicides, herbicides, and plant growth-regulating agents. Specific agents include those described, for example, in The Pesticide Manual, 14th edition, published by The British Crop Protection Council; and SHIBUYA INDEX, the 13th edition, 2008, published by SHIBUYA INDEX RESEARCH GROUP. Preferred other harmful organism control agents admixable into the compound of the present invention include insecticides and/or fungicides, more preferably insecticides. Fungicides are additionally admixable into insecticides.

Preferred examples of harmful organism control agents admixable into compounds represented by formula (I) or salts thereof according to the present invension include insecticides, for example, organic phosphoric ester compounds, carbamate compounds, nereistoxin derivatives, organochlorine compounds, pyrethroid compounds, benzoyl urea compounds, juvenile hormone analogies, molting hormone analogies, neonicotinoid compounds, sodium channel blockers for nerve cells, insecticidal macrocyclic lactones, γ-aminobutyric acid (GABA) antagonists, ryanodine receptor agonistic compounds, insecticidal ureas, BT agents, and entomopathogenic viral agents. More specific examples thereof include acephate, dichlorvos, EPN, fenitrothion, fenamifos, prothiofos, profenofos, pyraclofos, chlorpyrifos-methyl, chlorfenvinphos, demeton, ethion, malathion, coumaphos, isoxathion, fenthion, diazinon, thiodicarb, aldicarb, oxamyl, propoxur, carbaryl, fenobucarb, ethiofencarb, fenothiocarb, pirimicarb, carbofuran, carbosulfan, furathiocarb, hyquincarb, alanycarb, methomyl, benfuracarb, cartap, thiocyclam, bensultap, dicofol, tetradifon, acrinathrin, bifenthrin, cycloprothrin, cyfluthrin, dimefluthrin, empenthrin, fenfluthrin, fenpropathrin, imiprothrin, metofluthrin, permethrin, phenothrin, resmethrin, tefluthrin, tetramethrin,tralomethrin, transfluthrin, cypermethrin, deltamethrin, cyhalothrin, fenvalerate, fluvalinate, ethofenprox, flufenprox, halfenprox, silafluofen, cyromazine, diflubenzuron, teflubenzuron, flucycloxuron, flufenoxuron, hexaflumuron, lufenuron, novaluron, penfluron, triflumuron, chlorfluazuron, diafenthiuron, methoprene, fenoxycarb, pyriproxyfen, halofenozide, tebufenozide, methoxyfenozide, chromafenozide, dicyclanil, buprofezin, hexythiazox, amitraz, chlordimeform, pyridaben, fenpyroxymate, flufenerim, pyrimidifen, tebufenpyrad, tolfenpyrad, fluacrypyrim, acequinocyl, cyflumetofen, flubendiamide, ethiprole, fipronil, ethoxazole, imidacloprid, nitenpyram, clothianidin, acetamiprid, dinotefuran, thiacloprid, thiamethoxam, sulfoxaflor, pymetrozine, bifenazate, spirodiclofen, spiromesifen, flonicamid, chlorfenapyr, pyriproxyfene, indoxacarb, pyridalyl, spinosad, avermectin, milbemycin, azadirachtin, nicotine, rotenone, BT agents, entomopathogenic viral agents, emamectinbenzoate, spinetoram, pyrifluquinazon, chlorantraniliprole, cyenopyrafen, spirotetramat, lepimectin, metaflumizone, pyrafluprole, pyriprole, dimefluthrin, fenazaflor, hydramethylnon, triazamate, and compounds represented by formula (VII) described in Japanese Patent No. 4015182 and International Publication WO 2008/066153:

[Chemical formula 10]

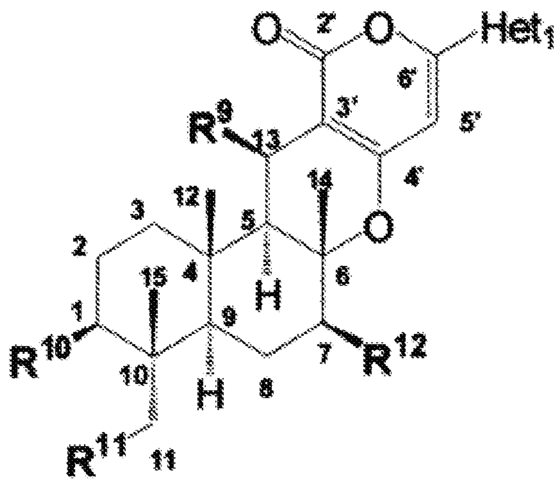

(VII)

wherein $Het_1$ represents 3-pyridyl; $R^9$ represents hydroxyl; $R^{10}$ and $R^{11}$ represent cyclopropylcarbonyloxy; and $R^{12}$ represents a hydrogen atom or hydroxyl.

Examples of more preferred other insecticides admixable into compounds represented by formula (I) or salts thereof according to the present invention include imidacloprid, nitenpyram, clothianidin, acetamiprid, dinotefuran, thiacloprid, thiamethoxam, and fipronil. Still more preferred examples thereof include clothianidin, imidacloprid, fipronil, dinotefuran, or compounds of formula (VII).

Examples of fungicides admixable into compounds represented by formula (I) or salts thereof according to the present invention include strobilrin compounds such as azoxystrobin, kresoxym-methyl, trifloxystrobin, orysastrobin, picoxystrobin, and fuoxastrobin, anilinopyrimidine compounds such as mepanipyrim, pyrimethanil, and cyprodinil, azole compounds such as triadimefon, bitertanol, triflumizole, etaconazole, propiconazole, penconazole, flusilazole, myclobutanil, cyproconazole, tebuconazole, hexaconazole, prochloraz, and simeconazole, quinoxaline compounds such as quinomethionate, dithiocarbamate compounds such as maneb, zineb, mancozeb, polycarbamate, and propineb, phenylcarbamate compounds such as diethofencarb, organochlorine compounds such as chlorothalonil and quintozene, benzimedazole compounds such as benomyl, thiophanate-methyl, and carbendazole, phenylamide compounds such as metalaxyl, oxadixyl, ofurase, benalaxyl, furalaxyl, and cyprofuram, sulfenic acid compounds such as dichlofluanid, copper compounds such as copper hydroxide and oxine-copper, isoxazole compounds such as hydroxyisoxazole, organophosphorus compounds such as fosetyl-aluminium and tolclofos-methyl, N-halogenothioalkyl compounds such as captan, captafol, and folpet, dicarboxyimide compounds such as procymidone, iprodione, and vinchlozolin, acid amide compounds such as thifluzamide and furametpyr, benzanilide compounds such as flutolanil and mepronil, morpholine compounds such as fenpropimorph and dimethomorph, organotin compounds such as fenthin hydroxide and fenthin acetate, cyanopyrrole compounds such as fludioxonil and fenpiclonil, and other fthalide, probenazole, acibenzolar-S-methyl, tiadinil, isotianil, carpropamid, diclocymet fenoxanil, tricyclazole, pyroquilon, tebufloquin, ferimzone, fluazinam, cymoxanil, triforine, pyrifenox, fenarimol, fenpropidin, pencycuron, cyazofamid, cyflufenamid, boscalid, penthiopyrad, proquinazid, quinoxyfen, famoxadone, fenamidone, iprovalicarb, benthiavalicarb-isopropyl, fluopicolide, pyribencarb, kasugamycin and validamycin.

Examples of more preferred fungicides admixable into compounds represented by formula (I) or salts thereof according to the present invention include orysastrobin, thifluzamide, furametpyr, fthalide, probenazole, acibenzolar-S-methyl, tiadinil, isotianil, carpropamid, diclocymet, fenoxanil, tricyclazole, pyroquilon, tebufloquin, and ferimzone. Still more preferred examples thereof include probenazole.

According to a preferred aspect of the present invention, there is provided a composition for use in controlling harmful organisms, comprising as an active ingredient at least one of compounds according to the present invention or salts thereof.

According to another preferred embodiment of the present invention, the composition for use in controlling harmful organisms according to the present invention is a composition for agricultural and horticultural insect pests.

According to another preferred aspect of the present invention, there is provided use of compounds according to the present invention or salts thereof as a harmful organism control agent.

According to another preferred aspect of the present invention, there is provided use of at least one of compounds according to the present invention or salts thereof as an agricultural and horticultural insect pest control agent.

Preferred examples of other harmful organism control agents admixable into compounds represented by formula (I) or salts thereof according to the present invention include organic phosphoric ester compounds, carbamate compounds, nereistoxin derivatives, organochlorine compounds, pyrethroid compounds, benzoyl urea compounds, juvenile hormone analogies, molting hormone analogies, neonicotinoid compounds, sodium channel blockers for nerve cells, insecticidal macrocyclic lactones, γ-aminobutyric acid (GABA) antagonists, ryanodine receptor agonistic compounds, insecticidal ureas, BT agents, entomopathogenic viral agents, polyether antibiotics, thiamine antagonists, and sulfa drugs/folic acid antagonist compounding agents. More specific examples thereof include acephate, dichlorvos, EPN, fenitothion, fenamifos, prothiofos, profenofos, pyraclofos, chlorpyrifos-methyl, haloxon, coumaphos, malathion, dimpylate, naled, tetradifon, diazinon, methomyl, thiodicarb, aldicarb, oxamyl, propoxur, carbaryl, fenobucarb, ethiofencarb, fenothiocarb, pirimicarb, carbofuran, benfuracarb, cartap, thiocyclam, dicofol, tetradifon, permethrin, tefluthrin, cypermethrin, deltamethrin, cyhalothrin, fenvalerate, fluvalinate, ethofenprox, silafluofen, diflubenzuron, teflubenzuron, flufenoxuron, chlorfluazuron, methoprene, chromafenozide, buprofezin, hexythiazox, amitraz, chlordimeform, pyridaben, fenpyroxymate, pyrimidifen, tebufenpyrad, fluacrypyrim, acequinocyl, cyflumetofen, flubendiamide, ethiprole, fipronil, ethoxazole, imidacloprid, clothianidin, thiamethoxam, thiacloprid, sulfoxaflor, pymetrozine, bifenazate, spirodiclofen, spiromesifen, flonicamid, chlorfenapyr, pyriproxyfene, indoxacarb, pyridalyl, spinosad, avermectin, milbemycin, milbemycin oxime, maduramycin, BT agents, entomopathogenic viral agents, emamectinbenzoate, spinetoram, pyrifluquinazon, chlorantraniliprole, cyenopyrafen, spirotetramat, lepimectin, metaflumizone, pyrafluprole, pyriprole, dimefluthrin, fenazaflor, hydramethylnon, triazamate, ivermectin, selamectin, moxidectin, doramectin, eprinomectin, dinotefuran, nitenpyram, acetamiprid, allethrin, d·d-T allethrin, dl·d-T80 allethrin, pyrethrins, phenothrin, flumethrin, cyfluthrin, d·d-T80 prarethrin, phthalthrin, transfluthrine, resmethrin, cyphenothrin, pyrethrum extract, synepirin 222, synepirin 500, pyriproxyfene, lufenuron, deet, trichlofon, tetrachlorvinphos, bromofenofos, cythioate, metoxadiazon, cyromazine, triflumuron, star anise oil, triclabendazole, flubendazole, fenbendazole, parbendazole, tiabendazole, antimony sodium gluconate, levamisole hydrochloride, bithionol, dichlorofen, bromopropylate, piperonylbutoxide, phenothiazine, piperazine carbon bisulfide, piperazine phosphate, piperazine adipate, piperazine citrate, melarsomine dihydrochloride, metyridine, santonin, pyrantel pamoate, pyrantel, morantel, praziquantel, febantel, emodepside, hygtomycin B, destomycin A, clorsulon, nitroxynil, diamfenethide, antimony sodium gluconate, levamisole hydrochloride, melrsonyl, dithiazanine iodide, stibophen, disophenol, dietylcarbamazine, diminazene, acrinamine, metronidazole, santonin, bunamidine, arecoline, and compounds represented by formula (VII) or agriculturally and horticulturally acceptable acid addition salts thereof.

According to another aspect of the present invention, there is provided a composition for use in controlling harmful organisms that comprises, in addition to the above ingredients, a suitable agriculturally and zootechnically acceptable carrier. The composition may be formulated into any suitable dosage forms, for example, emulsifiable concentrates, liquid formulations, suspensions, wettable powders, water dispersible granules, flowables, dusts, DL dusts, granules, micro granule fines, tablets, oils, aerosols, fumigants or microcapsules. These dosage forms may be produced as described, for example, in "Noyaku Seizai Gaido (Guide for Pesticide Formulation)" edited by "Pesticide Science Society of Japan/Seyoho Kenkyukai (Special Committee on Agricultural Formulation and Application)", Japan Plant Protection Association, 1997.

Carriers usable herein include solid carriers, liquid carriers, gaseous carriers, surfactants, dispersants and other adjuvants for formulations.

Solid carriers include, for example, talc, bentonite, clay, kaolin, diatomaceous earth, vermiculite, white carbon and calcium carbonate.

Liquid carriers include, for example, alcohols such as methanol, n-hexanol and ethylene glycol; ketones such as acetone, methyl ethyl ketone and cyclohexanone; aliphatic hydrocarbons such as n-hexane, kerosine and kerosene; aromatic hydrocarbons such as toluene, xylene and methyl naphthalene; ethers such as diethyl ether, dioxane and tetrahydrofuran; esters such as ethyl acetate; nitriles such as acetonitrile and isobutyronitrile; acid amides such as dimethylformamide and dimethylacetamide; vegetable oils such as soybean oil and cotton seed oil; dimethylsulfoxide; and water.

Gaseous carriers include, for example, LPG, air, nitrogen, carbon dioxide and dimethyl ether.

Surfactants or dispersants usable for emulsifying, dispersing or spreading include, for example, alkylsulfuric esters, alkyl (aryl) sulfonic acid salts, polyoxyalkylene alkyl (aryl) ethers, polyhydric alcohol esters and lignin sulfonic acid salts.

Adjuvants usable for improving the properties of formulations include, for example, carboxymethylcellulose, gum arabic, polyethylene glycol and calcium stearate.

The above carriers, surfactants, dispersants and adjuvants may be used either solely or in a combination according to need.

The content of the active ingredient in the comnposition of the present invention in terms of the content of the

active ingredient in these formulations is appropriately generally 1 to 75% by weight for emulsifiable concentrate, generally 0.3 to 25% by weight for dust, generally 1 to 90% by weight for wettable powder, and generally 0.5 to 10% by weight for granules.

The above formulations as such may be used, if necessary after dilution, for application directly to object insect pests or for application to plants, seeds, soil and the like. Preferably, the formulations may be used for application to seeds and soil.

According to a preferred aspect of the present invention, there is provided a method for controlling agricultural and horticultural insect pests, which comprises applying an effective amount of a composition for use in controlling harmful organisms, particularly a composition for use in controlling harmful organisms adapted for agricultural and horticultural insect pests according to the present invention to an object selected from the group consisting of water surface, soil, nutrient solution in nutriculture, solid medium in nutriculture, and seed, root, tuber, bulb, and rhizome of a plant.

According to another preferred aspect of the present invention, there is provided a method for controlling agricultural and horticultural insect pests, which comprises applying an effective amount of a composition for use in controlling harmful organisms, particularly a composition for use in controlling harmful organisms adapted for agricultural and horticultural insect pests according to the present invention to a plant.

Preferred methods for applying an effective amount of the composition for use in controlling harmful organisms, particularly the composition for use in controlling harmful organisms adapted for agricultural and horticultural insect pests include spreading treatment, soil treatment (such as mixing or irrigation), surface treatment (coating, dust coating, or covering), or fumigation treatment. More preferred are soil treatment and surface treatment.

[EXAMPLES]

The present invention is further illustrated by the following Examples that are not intended as a limitation of the invention.

<Synthesis Examples>

Synthesis Example 1 (Compound 1)

[Chemical formula 11]

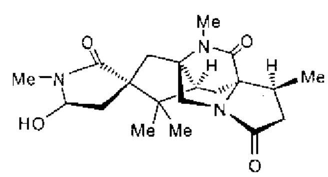

16-Keto aspergillimide (50 mg) was dissolved in 1.0 ml of THF, and 1.0 ml of methanol was added to the solution. $NaBH_4$ (25 mg) was added thereto, and the mixture was stirred at room temperature for one min. Further, 25 mg of $NaBH_4$ was added, and the disappearance of the starting material was confirmed. An aqueous ammonium chloride solution was added to the reaction solution, and the mixture was extracted with chloroform. The extract was dried over anhydrous sodium sulfate, and the solvent was removed by evaporation under the reduced pressure. The crude compound thus obtained was subjected to preparative TLC ($CHCl_3$ : MeOH = 10 : 1) to give compound 1 (40 mg, 80%).

$^1$H NMR($CDCl_3$) 0.93 (3H, s), 0.95 (3H, s), 1.45 (3H, d, J = 6.5 Hz), 1.45 (1H, m), 1.53 (1H, d, J = 6.5 Hz), 2.04-2.16 (2H, m), 2.20-2.32 (2H, m), 2.43 (1H, dd,J= 7.5, 16.0 Hz), 2.66 (1H, d, J = 15.0 Hz), 2.76(1H, d, J = 16.5 Hz), 2.80 (3H, s), 2.87 (3H, s), 2.93 (1H, br t, J = 9.5 Hz), 3.26(1H, d, J = 12.5 Hz), 3.30(1H, d, J = 12.0 Hz), 3.90 (1H, br s), 5.00 (1H, br s); MS (FAB) m/z 376 $(M+H)^+$

Synthesis Example 2 (Compound 2 (1 : 4))

[Chemical formula 12]

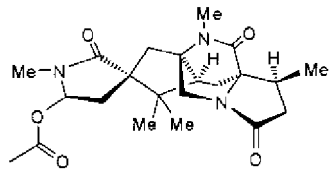

Compound 1 (15.0 mg) obtained in Synthesis Example 1 was dissolved in 1.0 ml of methylene chloride, 33.0 μl of pyridine and 15.0 mg of 4-DMAP were added to the solution. Acetyl chloride (7.0 μl) was added thereto, and the mixture was stirred at room temperature for one hr. Acetyl chloride (7.0 μl) was added thereto, and the mixture was stirred at room temperature all night. An aqueous ammonium chloride solution was added to the reaction solution, and the mixture was then extracted with chloroform. The organic layer was washed with sodium bicarbonate water and brine, and was then dried over anhydrous sodium sulfate. The solvent was removed by evaporation under the reduced pressure to give compound 2 (isomer ratio = 1 : 4) (15.0 mg, 90%). $^1$H NMR($CDCl_3$) 0.88, 0.92, 0.99 & 1.00 (6H, each s), 1.50 (3H, d, J = 6.4 Hz), 1.58 (1H, d, J = 15.1Hz), 1.70 & 1.82 (1H, each d, J = 15.8Hz), 2.10 (3H, s), 2.05-2.60 (4H, m), 2.62-3.00 (6H, m), 6.02 & 6.10 (1H, each br d, J = 6.1 Hz);
MS (FAB) m/z 418 $(M+H)^+$

Synthesis Example 3 (Compound 3)

[Chemical formula 13]

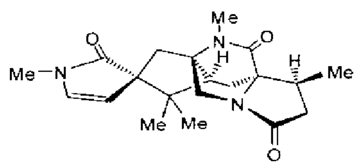

Compound 1 (20 mg) obtained in Synthesis Example 1 was dissolved in 1 ml of methylene chloride, and 44 μl of pyridine and 20 mg of 4-DMAP were added to the solution. Acetyl chloride (14 μl) was added thereto, and the mixture was stirred at room temperature for one hr. Further, acetyl chloride (14 μl) was added thereto, and the mixture was stirred at room temperature all night. An aqueous ammonium chloride solution was added to the reaction solution, and the mixture was then extracted with chloroform. The organic layer was washed with sodium bicarbonate water and brine, and was then dried over anhydrous sodium sulfate. The solvent was removed by evaporation under the reduced pressure. The crude compound thus obtained was purified by preparative TLC ($CHCl_3$ : MeOH = 10 : 1) to give compound 3 (10 mg, 53%). $^1$H NMR ($CDCl_3$) 0.82 (3H, s), 1.01 (3H, s), 1.49(1H, m) 1.51 (3H, d, J = 7.0 Hz), 1.85 (1H, d, J = 15.5 Hz), 2.08-2.18 (1H, m), 2.25-2.35 (1H, m), 2.38(1H, dd,J = 11.5, 16.0 Hz), 2.47 (1H, d, J = 15.5 Hz), 2.50 (1H, dd, J = 8.0 Hz, 16.0 Hz), 2.95 (3H, s), 2.98 (3H, s), 3.08 (1H, br t, J = 10.0 Hz), 3.33-3.47 (1H, m), 5.23 (1H, d, J = 4.8 Hz), 6.42 (1H, d, J = 4.8 Hz).;
MS (FAB) m/z 358$(M+H)^+$

Synthesis Example 4 (Compound 4)

[Chemical formula 14]

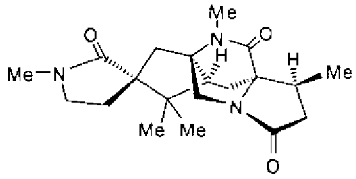

Compound 3 (13.0 mg) obtained in Synthesis Example 3 was dissolved in 1.0 ml of ethanol, and 5.0 mg of 10% Pd-C was added to the solution. A balloon filled with hydrogen gas was attached, and the mixture was stirred at room temperature all night. The reaction solution was filtered to remove 10% Pd-C. The reaction solution was removed by evaporation under the reduced pressure to give compound 4 (13.0 mg, 100%).

$^1$H NMR($CDCl_3$) 0.90 (3H, s), 1.00 (3H, s), 1.46(1H, dd, J = 10.2, 13.0 Hz)1.49(3H, d, J = 6.8 Hz), 1.55 (1H, d, J = 15.0 Hz), 1.86 (1H, t, J = 8.3 Hz), 1.90 (1H, t, J = 8.3 Hz), 2.10 (1H, dd, J = 10.7, 12.7 Hz), 2.30 (1H, m), 2.35(1H, dd,J = 10.7, 16.1 Hz), 2.48 (1H, dd, J = 7.8 Hz, 16.1 Hz), 2.73 (1H, d, J = 15.1 Hz), 2.85 (3H, s), 2.93 (3H, s), 3.17-3.32 (2H, m), 3.32 (1H, d, J = 12.0 Hz), 3.36 (1H, d, J = 12.0 Hz).; MS (FAB) m/z 360$(M+H)^+$

Synthesis Example 5 (Compound 5)

[Chemical formula 15]

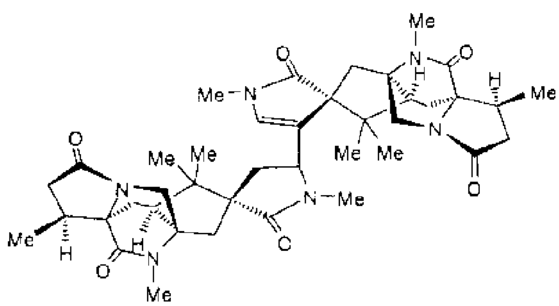

Compound 1 (10 mg) obtained in Synthesis Example 1 was dissolved in 500 μl of methylene chloride, and 10 mg of 4-DMAP was then added to the solution. The mixture was stirred at 0°C for 10 min. Trifluoromethanesulfonic acid anhydride (5.5 μl) was added thereto, and

the mixture was stirred at room temperature for one hr. Trifluoromethanesulfonic acid anhydride (5.5 μl) was added thereto, and

the mixture was stirred at room temperature all night. An aqueous ammonium chloride solution was added to the reaction solution, and the mixture was extracted with chloroform. The organic layer was washed with sodium bicarbonate water and brine, and was dried over anhydrous sodium sulfate. The solvent was removed by evaporation under the reduced pressure. The crude compound thus obtained was purified by preparative TLC ($CHCl_3$ : MeOH = 10 : 1) to give compound 5 (5.5 mg, 29%).

$^1$H NMR($CDCl_3$) 0.84 (3H, s), 0.97 (3H, s), 1.05 (3H, s), 1.09 (3H, s), 1.51 (3H,d, J = 7.0 Hz), 1.55 (3H, d, J = 7.0 Hz), 1.80 (1H, dd, J = 9.0, 13.5 Hz), 1.87(1H, d, J = 15.5 Hz), 2.13 (1H, dd, J = 10.6, 12.9 Hz), 2.16 (1H, dd, J = 10.6,12.9 Hz), 2.25-2.43 (4H, m), 2.47-2.55 (3H, m), 2.65 (3H, s), 2.79 (1H, br t, J= 10.0 Hz), 2.88 (1H, dd, J = 5.5, 13.5 Hz), 2.95 (1H, d, J = 15.5 Hz), 3.00 (3H, s), 3.01 (3H, s), 3.27 (3H, s), 3.29 (1H, br t, J = 10.0 Hz), 3.35 (1H, s), 3.48 (1H, dd, J = 12.0, 18.0 Hz), 3.90 (1H, dd, J = 6.0, 9.0 Hz).;

MS (FAB) m/z 715$(M+H)^+$

Synthesis Example 6 (Compound 6)

[Chemical formula 16]

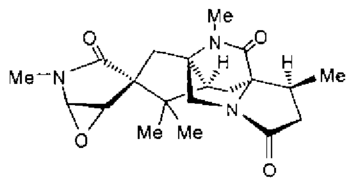

Compound 3 (9.0 mg) obtained in Synthesis Example 3 was dissolved in 1.0 ml of methylene chloride. m-CPBA (8.7 mg) was added to the solution, and the mixture was stirred at 60˚C all night. A 10% aqueous hypo solution was added to the reaction solution, and the mixture was extracted with chloroform. The organic layer was washed with sodium bicarbonate water and brine, and was then dried over anhydrous sodium sulfate. The solvent was removed by evaporation under the reduced pressure to give compound 6 (9.0 mg, 96%).
$^1$H NMR($CDCl_3$) 0.86 (3H, s), 1.02 (3H, s), 1.48 and 1.51 (3H, each s), 2.92 (3H,s), 2.97 (3H, s), 5.03 and 5.07 (1H, each m).;
MS (FAB) m/z 374 $(M+H)^+$

Synthesis Example 7 (Compound 7)

[Chemical formula 17]

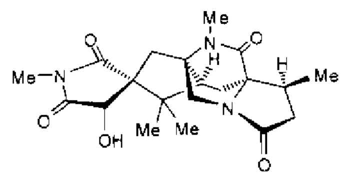

Compound 3 (9.0 mg) obtained in Synthesis Example 3 was dissolved in 1.0 ml of acetone and 0.5 ml of water, and 22.0 mg of metaperiodic acid was added to the solution. An aqueous potassium permanganate solution (0.5 ml, 0.02 mol) was then added dropwise thereto, and the mixture was stirred at room temperature all night. A 10% aqueous hypo solution was added to the reaction solution, and the mixture was extracted with chloroform. The organic layer was washed with sodium bicarbonate water and brine, and was then dried over anhydrous sodium sulfate. The solvent was removed by evaporation under the reduced pressure to give compound 7 (3.5 mg, 36%) .
$^1$H NMR($CDCl_3$) 0.99 (3H, s), 1.12 (3H, s), 1.55 (3H, d, J = 6.5 Hz), 2.15-2.25 (2H, m), 2.26-2.80 (4H, m), 2.90 (3H, s), 3.02 (3H, s), 3.11 (1H, m), 3.44(1H, br s), 4.42(1H, br s).; MS (FAB) m/z 390 $(M+H)^+$

Synthesis Example 8 (Compound 8 (1 : 1))

[Chemical formula 18]

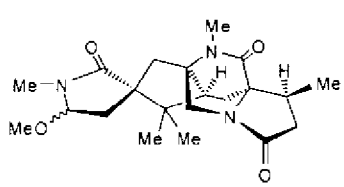

Compound 1 (8.0 mg) obtained in Synthesis Example 1 was dissolved in 500 μl of THF, and 2.5 mg of sodium hydride was added to the solution. Methyl iodide (6.5 μl) was then added thereto, and the mixture was stirred at room temperature for one hr. An aqueous ammonium chloride solution was added to the reaction solution, and the mixture was extracted

with chloroform. The extract was dried over anhydrous sodium sulfate. The solvent was then removed by evaporation under the reduced pressure to give compound 8 (isomer ratio = 1 : 1) (8.0 mg, 94%).

$^1$H NMR($CDCl_3$) 0.85, 0.92, 0.95 & 0.98 (6H, each s), 1.42-1.55 (4H, m), 1.63-1.70(1H, m), 1.78(0.5H, dd, J = 1.4, 13.9 Hz), 1.95(0.5H, dd, J = 6.1, 14.4 Hz), 2.07-2.17(1H, m), 2.22-2.53(4H, m), 2.68 & 2.77 (1H, each d, J = 15.0 Hz), 2.83-2.95 (6.5H, m), 3.20 (0.5H, br t, J = 10.5 Hz), 3.35 & 3.39 (3H, each s), 4.54(0.5H, br d, J = 5.4 Hz), 4.63(0.5H, dd, J = 1.7, 6.0 Hz).; MS (FAB) m/z 390 $(M+H)^+$

Synthesis Examples 9 (Compound 9)

[Chemical formula 19]

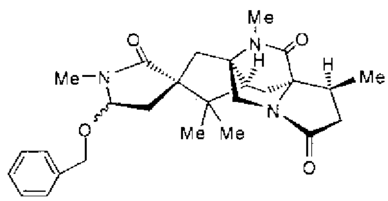

Compound 1 (8.0 mg) obtained in Synthesis Example 1 was dissolved in 500 μl of DMF, and 1.7 mg of sodium hydride was added to the solution. Benzyl bromide (5.5 μl) was added thereto, and the mixture was stirred at room temperature for 4 hr. An aqueous ammonium chloride solution was added to the reaction solution, and the mixture was extracted with chloroform. The extract was dried over anhydrous sodium sulfate. The solvent was then removed by evaporation under the reduced pressure. The crude compound thus obtained was purified by preparative TLC ($CHCl_3$ : MeOH = 20 : 1) to give compound 9 (6.0 mg, 61%).

$^1$H NMR ($CDCl_3$) 0.86 (3H, s), 0.91 (3H, s), 1.46 (3H, dd, J = 10.2 Hz, 12.2 Hz), 1.50 (1H, d, J = 6.4 Hz), 1.68 (1H, d, J = 16.1 Hz), 1.82 (1H, dd, J = 1.5, 14.2Hz), 2.12 (1H, dd, J = 9.3, 11.7 Hz), 2.22-2.32 (2H, m), 2.36 (1H, dd, J = 12.2Hz, 15.6 Hz), 2.48 (1H, dd, J = 7.8 Hz, 15.6 Hz), 2.73 (1H, d, J = 15.6 Hz), 2.83 (3H, s), 2.89 (3H, s), 3.20 (1H, br t, J = 10.5 Hz), 4.59(1H, dd, J = 12.0,17.1 Hz), 4.82(1H, dd, J = 1.7, 5.6 Hz), 7.26-7.28(5H, m).; MS (FAB) m/z 466$(M+H)^+$

Synthesis Example 10 (Compound 10)

[Chemical formula 20]

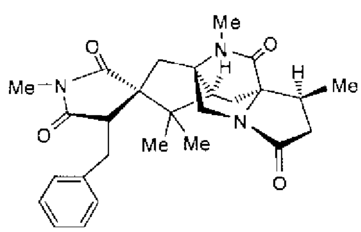

16-Keto aspergillimide (15 mg) was dissolved in 500 μl of DMF, and 2.7 mg of sodium hydride was added to the solution. The mixture was stirred at room temperature for 10 min, 9.6 μl of benzyl bromide was added thereto, and the mixture was stirred for 30 min. An aqueous ammonium chloride solution was added to the reaction solution, and the mixture was then extracted with chloroform. The extract was dried over anhydrous sodium sulfate. The solvent was removed by evaporation under the reduced pressure. The crude compound thus obtained was purified by preparative TLC ($CHCl_3$: MeOH = 10:1) to give compound 10 (6 mg, 48%).

$^1$H NMR ($CDCl_3$) 0.78 (3H, s), 0.80 (3H, s), 1.41 (1H, dd, J = 12.5 Hz, 15.5 Hz),1.50 (3H, d, J = 7.0 Hz), 2.02 (1H, d, J = 15.5 Hz), 2.09 (1H, dd, J = 10.6, 12.9Hz), 2.20-2.27 (1H, m), 2.35 (1H, dd, J = 11.8 Hz, 16.5 Hz), 2.47 (1H, dd, J = 7.9, 16.5 Hz), 2.52 (1H, d, J = 15.5 Hz), 2.83(1H, dd, J = 8.6, 14.5 Hz), 2.88-2.96 (2H, m), 2.97 (3H, s), 2.99 (3H, s), 3.13-3.17 (1H, m), 3.20-3.24 (2H, m),7.19-7.35 (5H, m).;

MS (FAB) m/z $464(M+H)^+$

<Preparation Examples>

Preparation Example 1 to 8 are Preparation Examples for agricultural and horticultural insect pest control, and Preparation Example 9 is Preparation Example for ectoparasite control.

Preparation Example 1 [Granules]

| | |
|---|---|
| Compound 8 | 5 wt% |
| Bentonite | 40 wt% |
| Talc | 10 wt% |
| Clay | 43 wt% |
| Calcium ligninsulfonate | 2 wt% |

The above ingredients were homogeneously ground and homogeneously mixed together. Water was added to the mixture, followed by thorough kneading. Thereafter, the kneaded product was granulated and dried to prepare granules.

Preparation Example 2 [Wettable powder]

| | |
|---|---|
| Compound 8 | 30 wt% |
| Clay | 50 wt% |
| White carbon | 2 wt% |
| Diatomaceous earth | 13 wt% |
| Calcium ligninsulfonate | 4 wt% |
| Sodium lauryl sulfate | 1 wt% |

The above ingredients were homogeneously mixed together, and the mixture was ground to prepare wettable powder.

Preparation Example 3 [Water dispersible granules]

| | |
|---|---|
| Compound 8 | 30 wt% |
| Clay | 60 wt% |
| Dextrin | 5 wt% |
| Alkylmaleic acid copolymer | 4 wt% |
| Sodium lauryl sulfate | 1 wt% |

The above ingredients were homogeneously ground and homogeneously mixed together. Water was added to the mixture, followed by thorough kneading. Thereafter, the kneaded product was granulated and dried to prepare water dispersible granules.

Preparation Example 4 [Floables]

| | |
|---|---|
| Compound 8 | 25 wt% |
| POE polystyrylphenyl ether sulfate | 5 wt% |

(continued)

| | |
|---|---|
| Propylene glycol | 6 wt% |
| Bentonite | 1 wt% |
| 1% aqueous xanthan gum solution | 3 wt% |
| PRONAL EX-300 (Toho Chemical Industry Co., Ltd.) | 0.05 wt% |
| ADDAC 827 (K.I. Chemical Industry Co., Ltd.) | 0.02 wt% |
| Water | To 100 wt% |

All the above ingredients except for the 1% aqueous xanthan gum solution and a suitable amount of water were premixed together, and the mixture was then ground by a wet grinding mill. Thereafter, the 1% aqueous xanthan gum solution and the remaining water were added to the ground product to prepare 100 wt% floables.

Preparation Example 5 [Emulsifiable concentrate]

| | |
|---|---|
| Compound 8 | 15 wt% |
| N,N-dimethylformamide | 20 wt% |
| Solvesso 150 (Exxon Mobil Corporation) | 55 wt% |
| Polyoxyethylene alkyl aryl ether | 10 wt% |

The above ingredients were homogeneously mixed together and dissolved to prepare an emulsifiable concentrate.

Preparation Example 6 [Dust]

| | |
|---|---|
| Compound 8 | 2 wt% |
| Clay | 60 wt% |
| Talc | 37 wt% |
| Calcium stearate | 1 wt% |

The above ingredients were homogeneously mixed together to prepare dust.

Preparation Example 7 [DL dust]

| | |
|---|---|
| Compound 8 | 2 wt% |
| DL clay | 94.5 wt% |
| White carbon | 2 wt% |
| Calcium stearate | 1 wt% |
| Light liquid paraffin | 0.5 wt% |

The above ingredients were homogeneously mixed together to prepare DL dust.

Preparation Example 8 [Micro granules fine]

| | |
|---|---|
| Compound 8 | 2 wt% |

(continued)

| | |
|---|---|
| Carrier | 94 wt% |
| White carbon | 2 wt% |
| Hisol SAS-296 | 2 wt% |

The above ingredients were homogeneously mixed together to prepare micro granules.

Preparation Example 9 [Liquid drops]

| | |
|---|---|
| Compound A | 20 wt% |
| Diethylene glycol monoethyl ether | 66.7wt% |
| Ethanol | 13.3 wt% |

The above ingredients were homogeneously mixed together to prepare liquid drops.

Test Example 1: Tickcidal effect against Haemaphysalis longicornis

Acetone solution (30 μl) of 200 ppm of compounds 1 to 9 and 20 ppm of compounds A and B was placed in 4 mL-volume glass vials. The vials were placed on a shaker, and a dry film of the compound was formed on the inner wall of the vials. The vials were dried for 24 hr or longer. Larva ticks of Haemaphysalis longicornis (10 heads) were released in the vials, and the vials were lidded. The vials were then left to stand in a humidistat chamber (25(C, humidity 85%, fully darkened conditions). One days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

The results were as shown in Table 3.

[Table 3]

| Compound | Concentration (ppm) | Death rate after one day |
|---|---|---|
| 1 | 200 | 80 |
| 2 | 200 | 100 |
| 3 | 200 | 80 |
| 4 | 200 | 100 |
| 6 | 200 | 100 |
| 7 | 200 | 100 |
| 8 | 200 | 100 |
| 9 | 200 | 100 |
| A | 20 | 100 |
| B | 20 | 100 |

Test Example 2 (Fleacidal effect against Ctenocephalides felis)

Acetone solution (200 μL) of 6.3 ppm of compound A was dropped on a filter paper having a diameter of 4.0

cm, and the filter paper was air-dried at room temperature to evaporate acetone. The chemical compound-treated filter paper was tightly fitted into a lid (diameter: 3.7 cm) for a 200-mL vial. About 25 adults of Ctenocephalides felis one to 3 days after adult eclosion were released in the 200 mL-volume vial, and the vial was then sealed by the vial lid with the chemical compound-treated filter paper fitted thereinto. The vial was inverted so that Ctenocephalides felis came into contact with the filter paper and, in this state, was left to stand. Three days after the release, the larvae were observed for survival or death, and the death rate of larvae was calculated by the following equation. The test was triplicated.

$$\text{Death rate (\%)} = \{\text{number of dead larvae}/(\text{number of survived larvae} + \text{number of dead larvae})\} \times 100$$

The results were as shown in Table 4.

[Table 4]

| Compound | Concentration (ppm) | Death rate after three days |
|---|---|---|
| A | 6.3 | 73 |

As is apparent from Test Examples 1 and 2, compounds A and B exhibited ectoparasiticidal effect at a lower concentration than the concentration of, for example, compound B described in Japanese Patent Laid-Open publication No. 245383/1998 that exhibits insecticidal activity against agricultural insect pests and hygiene insect pests.

Test Example 3 (Single oral administration actute toxicity against rat)

Compound A was suspended at a concentration of 100 mg/mL using 0.5% methylcellulose. The test solution was forcibly orally administered to rats (Jcl: Wistar, female, 8 week olds) with a tube and a disposable syringe. The dose was 2000 mg/kg. The rats were observed for survival or death from after the administration to the elapse of 6 hr over time and from the first to 15 days after the administration. The results were as show in Table 5.

[Table 5]

| Compound | Dose (mg/kg) | Cumulative number of dead rats 15 days after administration (number of death/total number of rats tested) |
|---|---|---|
| A | 2000 | 0/3 |

$LD_{50}$ values of compound A for the rats were not less than 2000 mg/kg, indicating that compound A is low-toxic to the rats.

Test Example 4 (Insecticidal effect against Aphis gossypii)

A leaf disk having a diameter of 2.0 cm was cut out from a cucumber in pot culture, and test solutions adjusted to a 200 ppm concentration (50% aqueous acetone solution; 0.05% Tween 20 added) were applied to the leaf disk. The leaf disk was then air dried, and first instar larvae were released and were then allowed to stand in a humidistat chamber (light period 16 hr - dark period 8 hr) (25°C). Three days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

Compounds 1, 2, 3, 4, 5, 6, 7, 8, 9 and 10 had an insecticidal activity of not less than 80% for Aphis gossypii.

Test Examples 5 (Insecticidal effect against Frankliniella occidentalis

A leaf disk having a diameter of 2.8 cm was cut out from kidney bean in pot culture, and a test solutions adjusted

to a 100 ppm concentration (50% aqueous acetone solution; 0.05% Tween 20 added) were applied to the leaf disk. The leaf disk was then air dried, and first instar larvae were released and were then allowed to stand in a humidistat chamber (light period 16 hr - dark period 8 hr) (25°C). Six days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

Compound 8 had an insecticidal activity of not less than 80% for Frankliniella occidentalis.

Test Example 6 (Insecticidal effect against Laodelphax striatellus)

A test solution (200 μl) adjusted to a 100 ppm concentration (10% aqueous acetone solution) was applied to the root of wheat seedlings in water culture. After the absorption of the test solution from the root for 3 days, 10 second instar larvae of Laodelphax striatellus were released, and the wheat seedlings were then allowed to stand in a humidistat chamber (light period 16 hr - dark period 8 hr) (25°C). Three days after the release, the larvae were observed for survival or death, and the death rate of larvae was calculated by the following equation. The test was duplicated.

$$\text{Death rate (\%)} = \{\text{number of dead larvae}/(\text{number of survived larvae} + \text{number of dead larvae})\} \times 100$$

Compounds 1, 2, 3, 4, 6, 7, 8 and 9 had an insecticidal activity of 100% for Laodelphax striatellus.

Test Example 7 (Tickcidal effect against Haemaphysalis longicornis on body surface of mouse)

Skin hair on the back of mice (ICR, male, 5 week olds) was clipped in a diameter of about 2 cm, and a 15-mL polystyrene conical tube cut to have a height of about 1.5 cm was bonded to the body surface of the mice with an instant adhesive. Test solutions (20 (l) adjusted with a solvent (diethylene glycol monoethyl ether : ethanol = 75 : 15) were dropped on the body surface of mice within the bonded tube. After thorough drying, larvae born of Haemaphysalis longicornis (10 or more heads) were released within the tube, and the tube was lidded. Three days after the release, the larvae were observed for survival or death, and the death rate of larvae was calculated by the following equation. The test was hexaplicated.

$$\text{Death rate (\%)} = \{\text{number of dead larvae}/(\text{number of survived larvae} + \text{number of dead larvae})\} \times 100$$

The results are shown in Table 6.

[Table 6]

| Compound | Dose (μg/2cm$^2$) | Death rate after three days |
|---|---|---|
| A | 10 | 100 |
| | 6 | 93 |
| | 2 | 60 |

**Claims**

1. A composition for use in controlling ectoparasites, comprising as an active ingredient at least one of compounds represented by formula (I) or salts thereof:

[Chemical formula 1]

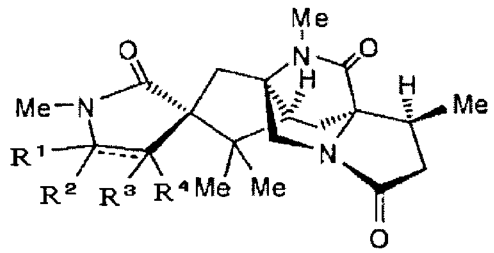

(I)

wherein
$R^1$ represents
a hydrogen atom,
hydroxyl,
optionally substituted $C_{1-10}$ alkyloxy,
optionally substituted $C_{7-15}$ aralkyloxy,
optionally substituted $C_{1-10}$ alkylcarbonyloxy, or
optionally substituted $C_{7-15}$ aralkylcarbonyloxy,
$R^2$ represents a hydrogen atom, or
$R^1$ and $R^2$ together represent oxo,
$R^3$ represents
a hydrogen atom,
hydroxyl,
optionally substituted $C_{1-5}$ alkyl, or
optionally substituted $C_{7-15}$ aralkyl,
$R^4$ represents a hydrogen atom,
or when $R^1$ and $R^3$ combine together to represent a double bond and
$R^2$ represents a hydrogen atom, $R^4$ represents formula (II)

[Chemical formula 2]

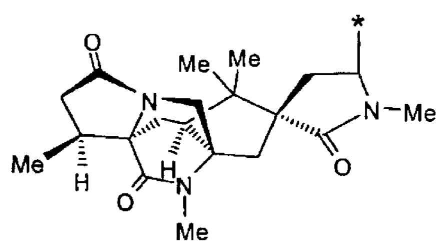

(II)

or when both $R^2$ and $R^4$ represent a hydrogen atom, $R^1$ and $R^3$ together form a cyclic ether, or $R^1$ and $R^3$ combine together to represent a double bond,
provided that
when $R^1$ represents hydroxyl,
optionally substituted $C_{1-10}$ alkyloxy,
optionally substituted $C_{7-15}$ aralkyloxy,
optionally substituted $C_{1-10}$ alkylcarbonyloxy, or
optionally substituted $C_{7-15}$ aralkylcarbonyloxy,

all of $R^2$, $R^3$ and $R^4$ then represent a hydrogen atom, or
when $R^1$ and $R^2$ together represent oxo, $R^3$ then represents hydroxyl, optionally substituted $C_{1-5}$ alkyl or optionally substituted $C_{7-15}$ aralkyl, or
compounds represented by formula (III):

[Chemical formula 3]

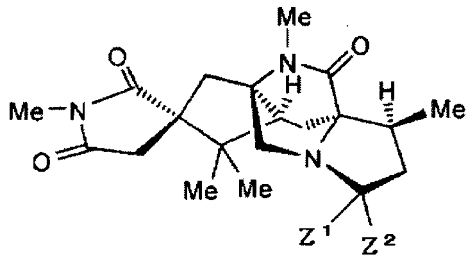

(III)

wherein $Z^1$ and $Z^2$ together represent oxo or each represent a hydrogen atom.

**2.** The composition according to claim 1, comprising as an active ingredient at least one of compounds represented by formula (IV) or salts thereof:

[Chemical formula 4]

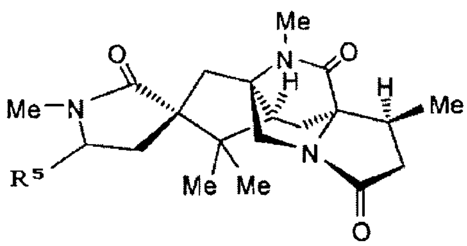

(IV)

wherein
$R^5$ represents
a hydrogen atom,
hydroxyl,
optionally substituted $C_{1-10}$ alkyloxy,
optionally substituted $C_{7-15}$ aralkyloxy,
optionally substituted $C_{1-10}$ alkylcarbonyloxy, or
optionally substituted $C_{7-15}$ aralkylcarbonyloxy.

**3.** The composition according to claim 1, comprising as an active ingredient at least one of compounds represented by formula (V) or salts thereof:

[Chemical formula 5]

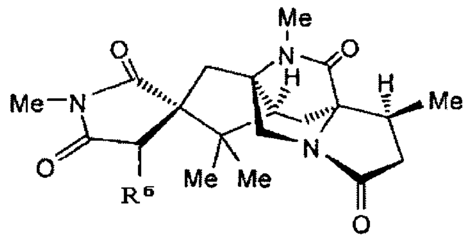

(V)

wherein $R^6$ represents hydroxyl, optionally substituted $C_{1-5}$ alkyl or optionally substituted $C_{7-15}$ aralkyl.

**4.** The composition according to claim 1, comprising as an active ingredient at least one of compounds represented by formula (VI) or salts thereof.

[Chemical formula 6]

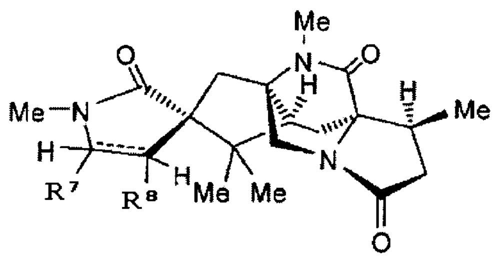

(VI)

wherein $R^7$ and $R^8$ together form a cyclic ether, or combine together to represent a double bond.

**5.** The composition according to claim 1, comprising as an active ingredient at least one of compounds represented by formula (I) or salts thereof
wherein all of $R^1$, $R^2$, $R^3$ and $R^4$ represent a hydrogen atom, or
$R^1$ represents hydroxyl, and $R^2$, $R^3$ and $R^4$ represent a hydrogen atom, or
$R^1$ and $R^2$ together represent oxo, and $R^3$ represents hydroxyl, and $R^4$ represents a hydrogen atom, or
$R^1$ and $R^2$ together represent oxo, $R^3$ represents benzyl, and $R^4$ represents a hydrogen atom, or
$R^1$ represents methoxy, and $R^2$, $R^3$ and $R^4$ represent a hydrogen atom, or
$R^1$ represents benzyloxy, and $R^2$, $R^3$ and $R^4$ represent a hydrogen atom, or
$R^1$ represents methylcarbonyloxy, and $R^2$, $R^3$ and $R^4$ represent a hydrogen atom, or
$R^1$ and $R^3$ combine together to represent a double bond, $R^2$ represents a hydrogen atom, and $R^4$ represents a group represented by formula (II).

**6.** The composition according to claim 1, comprising as an active ingredient at least one of compounds represented by formula (III) or salts thereof.

**7.** Use of at least one of compounds represented by formula (I) or (III) as defined in claim 1 or salts thereof as an ectoparasite control agent.

**8.** A method for controlling ectoparasites, comprising applying an effective amount of at least one of compounds represented by formula (I) or (III) as defined in claim 1 or salts thereof to an animal.

**9.** A compound represented by formula (I) as defined in claim 1 or a salt thereof.

**10.** A compound represented by formula (IV) as defined in claim 2 or a salt thereof.

**11.** A compound represented by formula (V) as defined in claim 3 or a salt thereof.

**12.** A compound represented by formula (VI) as defined in claim 4 or a salt thereof.

**13.** The compound according to claim 9, which is a compound represented by formula (I) as defined in claim 1 or a salt thereof, wherein
all of $R^1$, $R^2$, $R^3$ and $R^4$ represent a hydrogen atom, or
$R^1$ represents hydroxyl, and $R^2$, $R^3$ and $R^4$ represent a hydrogen atom, or
$R^1$ and $R^2$ together represent oxo, and $R^3$ represents hydroxyl, and $R^4$ represents a hydrogen atom, or
$R^1$ and $R^2$ together represent oxo, $R^3$ represents benzyl, and $R^4$ represents a hydrogen atom, or
$R^1$ represents methoxy, and $R^2$, $R^3$ and $R^4$ represent a hydrogen atom, or
$R^1$ represents benzyloxy, and $R^2$, $R^3$ and $R^4$ represent a hydrogen atom, or
$R^1$ represents methylcarbonyloxy, and $R^2$, $R^3$ and $R^4$ represent a hydrogen atom, or
$R^1$ and $R^3$ combine together to represent a double bond, and $R^4$ represents a group represented by formula (II) as defined in claim 1.

**14.** A composition for use in controlling harmful organisms, which comprises as an active ingredient at least one of compounds according to any one of claims 9 to 13 or salts thereof.

**15.** The composition according to claim 14, which is a composition for agricultural and horticultural insect pests.

**16.** Use of at least one of compounds according to any one of claims 9 to 13 or salts thereof as a harmful organism control agent.

**17.** Use of at least one of compounds according to any one of claims 9 to 13 or salts thereof as an agricultural and horticultural insect pest control agent.

**18.** A method for controlling agricultural and horticultural insect pests, which comprises applying an effective amount of a composition according to claim 14 or 15 to an object selected from the group consisting of water surface, soil, nutrient solution in nutriculture, solid medium in nutriculture, and seed, root, tuber, bulb, and rhizome of a plant.

**19.** A method for controlling agricultural and horticultural insect pests, which comprises applying an effective amount of a composition according to claim 14 or 15 to a plant.

**INTERNATIONAL SEARCH REPORT**

International application No.
PCT/JP2009/071257

A. CLASSIFICATION OF SUBJECT MATTER
*A01N43/90*(2006.01)i, *A01N63/04*(2006.01)i, *A01P7/04*(2006.01)i, *A61K31/529*(2006.01)i, *A61P33/14*(2006.01)i, *C07D471/22*(2006.01)i, *C07D491/22*(2006.01)i

According to International Patent Classification (IPC) or to both national classification and IPC

B. FIELDS SEARCHED

Minimum documentation searched (classification system followed by classification symbols)
A01N43/90, A01N63/04, A01P7/04, A61K31/529, A61P33/14, C07D471/22, C07D491/22

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2008/156165 A1 (Meiji Seika Kaisha, Ltd.), 24 December 2008 (24.12.2008), (Family: none) | 1,6-9,14-19 |
| X | Tennen Yuki Kagobutsu Toronkai Koen Yoshisyu, (1997), 39th, p.541-546 | 1,6-8 |
| X<br>A | JP 10-245383 A (Kumiai Chemical Industry Co., Ltd.), 14 September 1998 (14.09.1998), entire text (Family: none) | 1,6-8<br>2-5,9-19 |
| X | J.Antibiotics, (1997), 50(10), p.840-6 | 9,14-19 |
| X | Recent Research Development in Agricultural & Biological Chemistry, (1998), 2, p.511-25 | 1,6-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 March, 2010 (02.03.10) | 30 March, 2010 (30.03.10) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2009/071257

C (Continuation). DOCUMENTS CONSIDERED TO BE RELEVANT

| Category* | Citation of document, with indication, where appropriate, of the relevant passages | Relevant to claim No. |
|---|---|---|
| A | Tetrahedron Lett., (2004), 45(23), p.4489-93 | 1 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008323882 A
- JP 2009078607 A
- JP 10245383 A
- JP 4015182 B
- WO 2008066153 A


### Non-patent literature cited in the description


- *The Journal of Antibiotics,* 1997, vol. 50 (10), 840-846
- **Further.** *Tetrahedron Letters,* 1997, vol. 38 (32), 5655-5658
- *Bioscience, Biotechnology and Biochemistry,* 2000, vol. 64 (1), 111-115
- *Journal of veterinary pharmacology and therapeutics,* 2002, vol. 25 (4), 241-250
- The Pesticide Manual. The British Crop Protection Council
- *Tetrahedron Letters,* 1997, vol. 38 (32), 5655-5658
- SHIBUYA INDEX. SHIBUYA INDEX RESEARCH GROUP, 2008
- Noyaku Seizai Gaido. Japan Plant Protection Association, 1997